Europäisches Patentamt

European Patent Office ·

Office européen des brevets

(19)

(11) Publication number: **0 170 408**
**B1**

(12) EUROPEAN PATENT SPECIFICATION

(45) Date of publication of patent specification: **02.03.88**

(51) Int. Cl.⁴: **B 01 J 27/22**

(21) Application number: **85304587.0**

(22) Date of filing: **27.06.85**

(54) Iron carbide on titania surface modified with group VA oxides as Fischer-Tropsch catalysts.

(30) Priority: **29.06.84 US 626068**

(43) Date of publication of application: ·
**05.02.86 Bulletin 86/06**

(45) Publication of the grant of the patent:
**02.03.88 Bulletin 88/09**

(84) Designated Contracting States:
**DE GB NL**

(56) References cited:
**DE-B- 973 187**
**GB-A- 678 941**
**US-A-2 690 449**
**US-A-4 149 998**
**US-A-4 436 834**

(73) Proprietor: **Exxon Research and Engineering Company**
**P.O.Box 390 180 Park Avenue**
**Florham Park New Jersey 07932 (US)**

(72) Inventor: **Chersich, Claudio Cimatti**
**467 Palisade Avenue**
**Englewood Cliffs New Jersey 07632 (US)**
Inventor: **Fiato, Rocco Anthony**
**275 Country Club Lane**
**Scotch Plains New Jersey 07076 (US)**
Inventor: **Wachs, Israel Ephraim**
**340 Rolling Knolls Way**
**Bridgewater New Jersey 08807 (US)**

(74) Representative: **Somers, Harold Arnold et al**
**ESSO Engineering (Europe) Ltd. Patents & Licences Apex Tower High Street**
**New Malden Surrey KT3 4DJ (GB)**

Courier Press, Leamington Spa, England.

## Description

This invention relates to catalyst compositions of matter comprising iron carbide supported on a surface modified titania support. More particularly, this invention relates to Fischer-Tropsch catalyst compositions comprising iron carbide supported on a surface modified titania support, wherein said support comprises a surface modifying oxide of tantalum, vanadium, niobium or mixture thereof supported on the surface of said titania and wherein at least a portion of said surface modifying oxide is in a non-crystalline form.

The use of iron-titania mixtures as Fischer-Tropsch catalyst for converting mixtures of CO and $H_2$ to hydrocarbons is well-known to those skilled in the art. For example, US—A—2,543,327 discloses titania promoted iron oxide for Fischer-Tropsch synthesis wherein the iron oxide is in the form of naturally occurring magnetite and preferably as Alan Wood ore. In this disclosure a typical catalyst is shown as prepared by mixing about 13,600 grams of Alan Wood ore with 98 grams of titania and 216 grams of potassium carbonate used as a promoter. The ratio of hydrogen to carbon monoxide disclosed as being preferably at least 2/1 and the results show that the catalyst has relatively poor activity with a large selectivity towards the production of methane and very little selectivity towards the production of $C_2+$ hydrocarbons. That is, the Fischer-Tropsch product was primarily methane. Similarly, GB—A—1,512,743 also discloses a titania promoted, massive iron type of Fischer-Tropsch catalyst wherein iron oxide is mixed with titanium oxide, zinc oxide and potassium carbonate with the resulting mixture being sintered and then reduced for many hours at 500°C. Although this catalyst has relatively reasonably activity with regard to converson of the CO and $H_2$ mixture, the product was primarily (i.e., about 73%) olefinic, unsaturated $C_2/C_4$ hydrocarbons and with only about 10% of $C_2/C_4$ hydrocarbons or alkanes being produced. US—A—4,192,777 and US—A—4,154,751 while directed towards the use of potassium promoted Group VA metal cluster catalysts in Fischer-Tropsch synthesis reactions, suggest that iron supported on titania would be useful Fischer-Tropsch catalysts but do not disclose the preparation of same. In their examples, they disclose iron on various supports other than titania with the amount of iron on the support generally being less than about 5 percent. US—A—4,261,865 dscloses an iron titanate-alkali metal hydroxide catalyst for preparing alpha-olefins for mixtures of CO and $H_2$. That is, the catalyst is not iron supported on titania along with an alkali metal hydroxide but rather an iron titanate compound.

Another example of a titania-promoted massive iron catalyst for Fischer-Tropsch synthesis may be found in the Volume 17, No. 3—4 React. Kinet. Catal. Lett., pages 373—378, (1971) tiled "Hydrocondensation of $CO_2$ (CO) Over Supported Iron Catalysts". This article discloses an iron oxide, titania, alumina, copper oxide catalyst promoted with potassium. Similarly, in European patent application EP 0 071770 A2 Fischer-Tropsch catalysts are disclosed which include iron-titania catalysts wherein the iron to titania ratio can be greater than 1/10. The actual iron-titania catalyst is not an iron supported on titania catalyst but an iron/titania catalyst produced by a coprecipitation technique wherein the active iron catalytic component is distributed throughout a titanium oxide matrix. Thus, the resulting catalyst was not iron supported on titania but rather a bulk phase iron/titania mixture, which, when used for Fischer-Tropsch synthesis, produced predominantly olefins. The amount of olefins produced was generally greater than about 80% of the total hydrocarbon product.

With regard to iron/titania catalysts for Fischer-Tropsch wherein the iron is supported on titania, a 1982 article by Vannice, *Titania-Supported Metals as CO Hydrogenation Catalysts,* J. Catalysis, v. 74, p.199—202 (1982), discloses the use of an iron/titania catalyst for Fischer-Tropsch synthesis wherein the amount of iron, calculated as metallic iron, is 5 percent of the iron/titania composite and the catalyst shows extremely little activity for Fischer-Tropsch synthesis. An article by Reymond et al, *Influence of The Support or of an Additive on The Catalytic Activity in the Hydrocondensation of Carbon Monoxide by Iron Catalysts* in "Metal-Support and Metal-Additive Effects in Catalysts", B. Imelik et al (Eds), Elsevier, Netherlands, p.337—348 (1982), also discloses the use of iron/titania Fischer-Tropsch catalysts wherein the iron is supported on the titania.

USA 4,149,998 to Tauster et al relates to heterogenous catalysts consisting of Group VIII metals, including iron, dispersed of oxide carriers selected from the group consisting of Ti, V, Nb, Ta and mixtures thereof and zirconium titanate and $BaTiO_3$. However, there is no suggestion in this patent that the catalytic metal be dispersed on a surface modified titania.

It has now been discovered that catalysts comprising iron carbide supported on a surface modified titania support wherein said support comprises a surface modifying Group VA oxide of tantalum, niobium, vanadium and mixtures thereof supported on the surface of said titania and wherein at least a portion of said surface modifying oxide is in a non-crystalline form are useful catalysts for Fischer-Tropsch hydrocarbon synthesis. Moreover, Fischer-Tropsch reactions conducted with these catalysts have been found to result in increased olefin and decreased methane make compared to Fischer-Tropsch catalysts comprising iron supported on titania wherein the surface of the titania has not been modified with a Group VA modifying oxide. Further, the catalysts of this invention produce a greater amount of heavier products and exhibit superior catalyst maintenance than similar catalysts on titania whose surface has not been modified with a Group VA oxide.

In a preferred embodiment at least about 25 wt.% of the surface modifying oxide of tantalum, niobium, vanadium or mixture thereof present on the titania surface will be in a non-crystalline form. In a particularly preferred embodiment, the catalyst will be pretreated with CO at elevated temperature prior to use.

The term surface modified titania as used herein refers to titania whose surface has been modified by an oxide of niobium, vanadium, tantalum or mixtures thereof in an amount such that the modified support exhibits properties different from titania whose surface has not been modified and also different from bulk niobia, tantala, vanadia or mixtures thereof. Concomitantly, the final catalyst composition will exhibit properties different from iron carbide supported on unmodified titania or on bulk niobia, tantala, vanadia or a mixture thereof.

Thus, the catalyst support useful for preparing the catalysts of this invention comprise titania whose surface has been modified with an oxide of a Group VA metal (vanadium, niobium, tantalum or a mixture thereof). That is, the surface of the titania has been modified by an oxide of vanadium, niobium, tantalum or a mixture thereof in an amount such that the catalyst exhibits properties different from titania whose surface has not been modified and different from bulk oxides of vanadium, niobium, tantalum or a mixture thereof. Those skilled in the art know that the oxides of niobium, tantalum or a mixture thereof are crystalline in their bulk form. Thus, at least a portion of and preferably at least 25 wt.% of the Group VA metal oxide will be in a non-crystalline form. This will be accomplished if the metal oxide loading on the titania broadly ranges between about 0.5 to 25 wt.% of the total catalyst weight.

In the catalysts of this invention the iron carbide is supported on the surface modified titania. Consequently, the catalysts of this invention are prepared by a two-step sequential process wherein the surface modified titania support is prepared first, followed by depositing the iron carbide or iron carbide precursor on the support. Thus, in the first step an oxide or precursor thereof of a metal selected from the group consisting of niobium, tantalum, vanadium and mixture thereof is deposited on the titania to form either the surface modified support or, in the case of one or more precursors, a support precursor. The support precursor will then be calcined to oxidize the oxide precursor and form a support comprising titania whose surface has been modified by an oxide of a metal selected from the group consisting of niobium, tantalum, vanadium and mixture thereof wherein at least a portion of said surface modifying oxide is in a non-crystalline form.

The catalyst support precursors of this invention may be prepared by techniques well-known in the art, such as incipient wetness, impregnation, etc., the choice being left to the practitioner. When using the impregnation technique, the impregnating solution is contacted with the titania for a time sufficient to deposit the oxide precursor material onto the titania either by selective adsorption or alternatively, the excess solvent may be evaporated during drying leaving behind the precursor salt. If an impregnation or incipient wetness technique is used to prepare a support precursor of this invention, the transition metal oxide salt solution used may be aqueous or organic, the only requirement being that an adequate amount of precursor compound for the selected Group VA transition metal oxide or oxides be soluble in the solvent used in preparing this solution.

The support precursor composite will then normally be dried at temperatures ranging from about 50°—300°C to remove the excess solvent and, if necessary, decompose the salt if it is an organic salt to form a catalyst precursor. The support precursor composite is then converted into the surface modified titania support by calcining at temperatures of from about 150° to 800°C and preferably 300°—700°C in a suitable oxidizing atmosphere such as air, oxygen, etc. The time required to calcine the composite will, of course, depend on the temperature and in general will range from about 0.5—7 hours. Reducing atmospheres may also be used to decompose the transition metal oxide precursors, but the resulting composite will then require subsequent calcination to convert the reduced metal component to the oxide form.

The supports of this invention will generally have metal oxide loadings of from about 0.5 to 25 wt.% metal oxide on the titania based on the total support composition, preferably from about 1 to 15 wt.%, more preferably from about 2 to 10 wt.% based in the total support composition.

It is important to this invention that the iron carbide is supported on and not merely mixed with the surface modified titania support.

The catalyst will be prepared by depositing a suitable iron precursor component onto the surface modified titania support from a precursor solution using any of the well-known techniques such as incipient wetness, multiple impregnation, pore-filling etc., the choice being left to the convenience of the practitioner. As has heretobefore been stated, it is important for the iron precursor to be deposited onto the support as opposed to other methods for catalyst preparation such as co-precipitation or physical mixtures. After impregnation, the impregnate is dried to remove excess solvent and/or water therefrom. The dry impregnate can then be converted to a catalyst of this invention employing a number of different methods. In one method, the impregnate will be converted directly to a catalyst of this invention by contacting same with a CO containing reducing gas, preferably a reducing gas containing a mixture of CO and $H_2$. Thus, it will be appreciated to those skilled in the art that the catalyst of this invention can be formed from the impregnation in-situ in a Fischer-Tropsch hydrocarbon synthesis reactor. However, it is preferred to employ a sequential treatment first contacting the dry impregnate with an $H_2$ containing reducing gas that does not contain CO to reduce the impregnate, followed by contacting the reduced impregnate with CO or a CO containing gas such as a mixture of CO and $H_2$ to form the catalyst of this invention. As a practical matter, it may be commercially advantageous to form the catalyst of this invention by subjecting the impregnate to calcining to convert the supported iron precursor component to iron oxide, followed by subsequent reduction and formation of the catalyst of this invention.

Promoter metals such as potassium or other alkali metals may be added via impregnation, etc. before the composite is contacted with a reducing atmosphere and/or CO containing gas to form the catalyst of this invention. In general, the amount of promoter metal present will range from about 0.5 to 5 wt.% based on the amount of iron (calculated as $Fe_2O_3$) supported on the titania.

If one desires to obtain a catalyst of this invention via a supported iron oxide route, then the dry impregnate will be calcined in air or other suitable oxidizing atmosphere at a temperature of from about 120 to 300°C for a time sufficient to convert the supported iron precursor component to iron oxide. After the iron/surface modified titania impregnate has been calcined to convert the supported iron precursor compound to iron oxide, the iron oxide/titania composite, with or without one or more promoter metals, is reduced in a hydrocarbon-containing, net-reducing atmosphere at a temperature broadly ranging from about 300—500°C for a time sufficient to convert the iron oxide to metallic iron. It has been found that if one tries to reduce the iron oxide/titania composite at as temperature below about 300°C, (i.e., 250°C), the catalyst of this invention will not subsequently be formed.

Irrespective of the route one employs to form a catalyst of this invention, whether by reduction followed by contacting with CO, direct formation of the catalyst or through the supported iron oxide route, it is important not to contact the composite with a reducing gas at temperatures above about 500°C.

Reduction temperatures exceeding about 500°C will produce a catayst which exhibits relatively low CO hydrogenation activity with less than 50% of the $C_2+$ hydrocarbons being alkanes. Further, even at a 500°C reduction temperature a less effective catalyst will be produced if the reduction occurs for too long a time, i.e., about ten hours or more. Thus it will be appreciated that the temperature range for reducing the composite to form a catalyst cannot be critically quantified with any degree of precision inasmuch as there exists a time-temperature continuum for proper reduction.

In a preferred embodiment of this invention, the catalyst composite will first be reduced, followed by contacting with CO at temperatures ranging from about 200 to 500°C and preferably 200 to 400°C for a time sufficient to form a catalyst comprising iron carbide supported on the surface modified titania. It has been found that a CO treatment followed hydrogen reduction dramatically improves the activity of the catalyst for CO conversion with only slight changes in product selectivity. Iron carbide on the surface modified titania support will also be achieved by treating the calcined iron/support composite with a mixture of CO and $H_2$, but it is preferred to use the sequential treatment comprising hydrogen reduction followed by CO treatment. Further, when using this sequential treatment to produce a catalyst of this invention, it is preferred that the temperature used for the CO treatment be lower than that used for the hydrogenation reduction. Thus, in general the CO treatment will occur at a temperature of about 100 to 200°C lower than the temperature used for the hydrogen reduction.

It has also been discovered that, if a catalyst of this invention has been prepared by hydrogen reduction and then contacted in-situ, in a reactor, with a feedstream comprising a mixture of CO and $H_2$ to form a catalyst of this invention, the activity of the so formed catalyst will be substantially increased by reducing or eliminating the hydrogen content of the feedstream, raising the temperature in the reactor an additional 50 to 150°C for a short period of time (i.e., 3—5 hours), followed by reestablishing the original reaction conditions.

Predominantly $C_2+$ alkane hydrocarbons are produced from mixtures of CO and $H_2$ by contacting said mixtures with the catalyst of this invention at temperatures ranging from about 200 to 350°C and preferably from about 250—320°C. The reaction pressure will generally range from about 100—500 psig (6.9—34.5 bar) and more preferably from about 150—300 psig (10.3—20.7 bar), although pressures outside this range may be used if desired. However, if one goes too low in pressure i.e., <50 psig (<3.4 bar), catalyst activity will be greatly reduced and methane production will predominate. Upper pressure limits will generally be dictated by economic considerations. The $H_2$/CO mole ratio in the reaction zone will generally range from about 1/2 to 3/1, preferably from about 1/2 to 2/1 and still more preferably from about 1/2 to 1/1.

The invention will be more readily understood by reference to the following examples.

## Catalyst Support Preparation

Degussa P—25, a mixture of anatase and rutile titania, was used as the titania support. Both of the catalyst supports were prepared in a glove box in a nitrogen atmosphere to prevent decomposition of the transition metal oxide precursors. In all cases 10 grams of the P—25 titania powder were slurried in 100 $cm^3$ of ethanol to which was added the transition metal oxide precursor, with the resulting mixture stirred overnight, under flowing nitrogen, to evaporate the ethanol. Each dry mixture was then taken out of the glove box and 3 $cm^3$ of water added. The resulting mixture was stirred overnight in air, then the dry powder placed in a quartz boat and slowly heated in a 1/1 flowing mixture of $O_2$ in He up to 400°C. At 400°C the He flow was cut off and the powdered, catalyst support precursor then heated from 400 to 575°C in 100% $O_2$. Each sample of catalyst precursor was held at 575°C in the $O_2$ for two hours to calcine the precursor into a surface modified titania support of this invention.

The transition metal oxide precursors were obtained from Alfa, Inc. and were $Nb(C_2H_5O)_5$ and $VO(C_2H_5O)_3$. The amounts of niobia and vanadia precursors added to each slurry of 10 g P—25 in 100 $cm^3$ of ethanol were 2.5 and 0.46 grams, respectively. The resulting catalysts contained 10 wt.% niobia on titania and 2 wt.% vanadia on titania. The niobia and vanadia contents of the catalysts were expressed as niobium pentoxide and vanadium pentoxide.

Example 1

A 5-8 cm³ sample of catalyst, containing 4 wt.% Fe as elemental iron on the support, was loaded into a 3/8 inch (9.5 mm) O.D. 316 stainless steel tubular reactor. The system was then flushed with nitrogen at atmospheric pressure and then flushed with 90% $H_2$/10% $N_2$ at atmospheric pressure. The reactor was then heated to 500°C in flowing 90% $H_2$/10% $N_2$ (100 cm³/min) and maintained at these conditions for 5 hrs. After this, the reactor was cooled to the desired reaction temperature, 290—315°C, and the pressure increased to 300 psig (20.7 bar). The reducing gas was then replaced with 1/1 $H_2$/CO at a flow rate (standard hourly spaced velocity) of 500 V/V/hr. The exit gas from the reactor was fed into a gas chromatograph for on-line analysis of $C_1$—$C_{15}$ hydrocarbons, CO, $CO_2$ and $N_2$.

The results of experiments A—D are presented in Table 1. The runs can be prepared either at conditions for nearly equal conversion, run A vs. Run C and Run B vs. Run D, or at identical conditions, Run A vs. Run D. In all cases the vanadium containing system is found to generate lower levels of methane than the standard catalyst. Comparison of these catalysts at identical conditions, Run A vs. Run D at 350°C, also indicates that the vanadium containing system is more active. The modified $TiO_2$ catalyst of the present invention is clearly superior to the unmodified analogue for production of desired α-olefin products while minimizing the formation of unwanted methane.

TABLE I

| Run | 4 wt.% Iron on Titania | | 4 wt.% Iron on Titania Surface Modified With An Oxide of Vanadium | |
|---|---|---|---|---|
| | A | B | C | D |
| Temp.°C | 305 | 315 | 290 | 305 |
| % CO Conversion | 47 | 60 | 49 | 70 |
| Wt.% Selectivity ($CO_2$ Free) | | | | |
| $CH_4$ | 21.0 | 24.4 | 16.1 | 17.8 |
| $C_2^=$ | 0.4 | 0.6 | 2.7 | 2.8 |
| $C_2^°$ | 16.1 | 16.2 | 20.2 | 16.8 |
| $C_3^=$ | 11.7 | 9.7 | 16.6 | 18.6 |
| $C_3^°$ | 13.7 | 12.7 | 14.4 | 16.5 |
| $C_4^=$ | 2.5 | 3.0 | 1.9 | 2.0 |
| $C_4^°$ | 7.1 | 7.4 | 8.1 | 10.9 |
| $C_5^+$ | 27.5 | 26.0 | 20.0 | 14.6 |

Conditions: 1:1 $H_2$:CO, 500 v/v/hr, 300 psig (20.7 bar), pretreatment with $H_2$ at 500°C for 5 hr. $C_5^+$ determined by nitrogen internal standard method.

The activity and carbon number distributions for the unmodified Fe/$TiO_2$ and the V and Nb surface modified Fe/$TiO_2$ catalysts during Fischer-Tropsch synthesis are presented in Table 2. The addition of V and Nb affected the activity and selectivity of the Fe/$TiO_2$ and the V and Nb surface midified Fe/$TiO_2$ catalysts. The incorporation of V and Nb to the $TiO_2$ surface increased and decreased the conversion of CO, respectively. The stability of the modified catalysts was superior to that of the unmodified Fe/$TiO_2$ catalyst (less coking). The V and Nb modified Fe/$TiO_2$ catalysts substantially decreased the $CH_4$ yield and increased the $C_5^+$ yield. Whereas Fe/$TiO_2$ yields substantial amounts of paraffins (70—90% paraffins in hydrocarbon) the V and Nb modified Fe/$TiO_2$ catalysts produced substantial amounts of olefins and low amounts of paraffins. In addition, XRD analysis of the spent V and Nb modified Fe/$TiO_2$ catalysts did not show the presence of $FeTiO_3$ in the catalysts. Thus, the addition of V and Nb to the surface of the $TiO_2$ altered the Fe-

$TiO_2$ interaction and the nature of the products obtained from such a catalyst during Fischer-Tropsch synthesis.

TABLE 2

| | 4% Fe/$TiO_2$ | 4% Fe($TiO_2$ + V oxide) | 4% Fe($TiO_2$ + Nb oxide) |
|---|---|---|---|
| % CO Conversion | 27.0 | 34.3 | 20.1 |
| $C_1$ | 21.0 | 13.0 | 14.6 |
| $C_2$ | 17.0 | 21.5 | 15.0 |
| $C_3$ | 29.0 | 12.0 | 11.9 |
| $C_4$ | 9.0 | 8.0 | 6.0 |
| $C_5{}^+$ | 24.0 | 45.5 | 52.5 |

CONDITIONS: 270°C, 300 psig (20.7 bar), 500—600 V/V/M, $H_2$:CO=1

**Claims**

1. A catalyst comprising iron carbide supported on a surface modified titania support wherein said support comprises an oxide of a metal selected from niobium, vanadium, tantalum and mixtures thereof, supported on said titania wherein at least a portion of said supported oxide of niobium, vanadium, tantalum or a mixture thereof is in a non-crystalline form.

2. A catalyst according to claim 1 containing one or more alkali metal promoters.

3. A catalyst according to either of claims 1 and 2 wherein the amount of said iron carbide, calculated as iron, ranges from about 2 to 20 wt.% of the total catalyst composition.

4. A catalyst according to claim 3 wherein the amount of supported iron carbide, calculated as iron, ranges from about 4 to 10 wt.% of the total catalyst composition.

5. A catalyst according to any one of the preceding claims wherein at least about 25 wt.% of said supported oxide is non-crystalline.

6. A process for producing a catalyst comprising iron carbide supported on a surface modified titania support wherein said support comprises an oxide of a metal selected from niobium, vanadium, tantalum and mixtures thereof, supported on titania wherein at least a portion of said supported oxide is in a non-crystalline form, said process comprising the steps of:

(a) depositing iron on the surface modified titania support from a solution of iron precursor compound in an amount such that the final catalyst will contain supported iron in an amount of at least about 2 milligrams of iron, calculated as $Fe_2O_3$, per square meter of titania support surface;

(b) calcining the iron precursor supported on titania produced in step (a) at a temperature of from about 120 to 500°C for a time sufficient to decompose said iron precursor material and convert at least a portion of said supported iron to $Fe_2O_3$; and

(c) contacting said calcined composite formed in step (b) with hydrogen at a temperature of from between about 300-500°C for a time sufficient to convert at least a portion of said supported iron to a reduced composite; and

(d) contacting said reduced composite formed in (c) with CO at an elevated temperature of at least about 200°C for a time sufficient to form said catalyst.

7. A process according to claim 6 wherein said reduced composite is contacted with CO at a temperature broadly between about 200 to 500°C prior to use.

8. A process for producing hydrocarbons, including alkane hydrocarbons, from gaseous feed mixtures of CO and $H_2$ comprising contacting said feed, at a temperature ranging from about 200 to 350°C and for a time sufficient to convert at least a portion of said feed to alkane hydrocarbons, with a catalyst according to any one of claims 1 to 5.

**Patentansprüche**

1. Eisencarbid enthaltender Katalysator, der auf einen oberflächenmodifizierten Titandioxidträger aufgebracht ist, dadurch gekennzeichnet, daß der Träger ein Oxid eines Metalls ausgewählt aus Niob, Vanadin, Tantal und Mischungen derselben aufgebracht auf das Titandioxid enthält, wobei mindestens ein Teil des auf dem Träger befindlichen Oxids von Niob, Vanadin, Tantal oder Mischungen derselben in einer nichtkristallinen Form vorliegt.

2. Katalysator nach Anspruch 1, dadurch gekennzeichnet, daß er einen oder mehrere Alkalimetallbeschleuniger enthält.

3. Katalysator nach Anspruch 1 oder 2, dadurch gekennzeichnet, daß die Menge des Eisencarbids, berechnet als Eisen, im Bereich von etwa 2—20 Gew.-% bezogen auf die gesamte Katalysatorzusammensetzung liegt.

4. Katalysator nach Anspruch 3, dadurch gekennzeichnet, daß die Menge des Eisencarbids, berechnet als Eisen, im Bereich von etwa 4—10 Gew.-% bezogen auf die gesamte Katalysatorzusammensetzung liegt.

5. Katalysator nach einem der vorangehenden Ansprüche, dadurch gekennzeichnet, daß mindestens etwa 25 Gew.-% des auf dem Träger befindlichen Oxids nicht kristallin sind.

6. Verfahren zur Herstellung eines Katalysators, der Eisencarbid auf einem oberflächenmodifizierten Titandioxidträger enthält, wobei der Träger ein Oxid eines Metalls ausgewählt aus Niob, Vanadin, Tantal und Mischungen derselben aufgebracht auf Titandioxid enthält und mindestens ein Teil des auf dem Träger befindlichen Oxids in einer nichtkristallinen Form vorgliegt, gekennzeichnet durch folgende Verfahrensstufen:

(a) Aufbringung von Eisen auf den oberflächenmodifizierten Titandioxidträger aus einer Lösung einer Eisenvorläuferverbindung in einer solchen Menge, daß der fertige Katalysator Eisen in einer Menge von mindestens etwa 2 mg, berechnet als $Fe_2O_3$, je $m^2$ Titandioxidträgeroberfläche enthält;

(b) Calcinieren des in Stufe (a) hergestellten, auf Titandioxid aufgebrachten Eisenvorläufers bei einer Temperatur von etwa 120—500°C über einer ausreichenden Zeitraum, um das Eisenvorläufermaterial zu zersetzen und mindestens einen Teil des auf den Träger aufgebrachten Eisens in $Fe_2O_3$ umzuwandeln;

(c) Kontaktieren der calcinierten, in Stufe (b) gebildeten Zusammensetzung mit Wasserstoff bei einer Temperatur zwischen etwa 300 und 500°C über einen ausreichenden Zeitraum, um mindestens einen Teil des auf dem Träger befindlichen Eisens in eine reduzierte Zusammensetzung umzuwandeln, und

(d) Kontaktieren der reduzierten, in Stufe (c) gebildeten Zusammensetzung mit CO bei einer erhöhten Temperatur von mindestens etwa 200°C über einen ausreichenden Zeitraum, um den Katalysator zu bilden.

7. Verfahren nach Anspruch 6, dadurch gekennzeichnet, daß die reduzierte Zusammensetzung vor der Verwendung bei einer Temperatur, die grob zwischen etwa 200 und 500°C liegt, mit CO kontaktiert wird.

8. Verfahren zur Herstellung von Kohlenwasserstoffen einschließlich Alkankohlenwasserstoffen aus gasförmigen Einsatzmaterialmischungen aus CO und $H_2$, gekennzeichnet durch Kontaktieren des Einsatzmaterials bei einer Temperatur im Bereich von etwa 200—350°C und über einen ausreichend langen Zeitraum, um mindestens einen Teil des Einsatzmaterials in Alkankohlenwasserstoffe umzuwandeln, mit einem Katalysator gemäß einem der Ansprüche 1 bis 5.

**Revendications**

1. Catalyseur comprenant du carbure de fer supporté sur un support d'oxyde de titane modifié en surface, dans lequel ce support comprend un oxyde d'un métal choisi parmi le niobium, le vanadium, le tantale er les mélanges de ceux-ci supporté sur l'oxyde de titane, une portion au moins de l'oxyde de niobium, vanadium, tantale ou mélange de ceux-ci supporté étant sous forme non cristalline.

2. Catalyseur selon la revendication 1 contenant un ou plusiers promoteurs qui sont des métaux alcalins.

3. Catalyseur selon l'une des revendications 1 et 2 dans lequel la quantité de carbure de fer, calculée comme étant du fer, s'échelonne de 2 à 20% en poids du total de la composition de catalyseur.

4. Catalyseur selon la revendication 3 dans lequel la quantité de carbure de fer supporté, calculée comme étant du fer, s'échelonne d'environ 4 à 10% en poids du total de la composition de catalyseur.

5. Catalyseur selon l'une quelconque des revendications précédentes dans lequel au moins 25% en poids environ de l'oxyde supporté est non cristallin.

6. Procédé de fabrication d'un catalyseur comprenant du carbure de fer supporté sur un support d'oxyde de titane modifié en surface dans lequel ce support comprend un oxyde d'un métal choisi parmi le niobium, le vanadium, le tantale et les mélanges de ceux-ci, supporté sur de l'oxyde de titane, une portion au moins de l'oxyde supporté étant sous forme non cristalline, ce procédé comprenant les étapes qui consistent à:

(a) déposer du fer sur le support d'oxyde de titane modifié en surface à partir d'une solution d'un composé précurseur du fer, en quantité telle que le catalyseur final contienne du fer supporté en quantité d'au moins environ 2 mg de fer, calculé comme étant $Fe_2O_3$, par mètre carré de surface de support d'oxyde de titane;

(b) calciner le précurseur du fer supporté sur oxyde de titane produit dans l'étape (a) à une température d'environ 120 à 500°C pendant un temps suffisant pour décomposer cette substance précursuer du fer et convertir au moins une portion du fer supporté en $Fe_2O_3$; et

(c) mettre en contact le composite calciné formé dans l'étape (b) avec de l'hydrogène à une température allant d'environ 300 à 500°C pendant un temps suffisant pour convertir au moins une portion du fer supporté en un composite réduit; et

(d) mettre en contact le composite réduit formé dans l'étape (c) avec CO à une température élevée d'environ au moins 200°C pendant un temps suffisant pour former le catalyseur.

7. Procédé selon la revendication 6 dans lequel le composite réduit est mis en contact avec CO à une température allant en gros d'environ 200 à 500°C avant l'emploi.

8. Procédé de production d'hydrocarbures, comprenant des hydrocarbures alcanes, à partir de mélanges d'alimentation gazeux de CO et $H_2$ comprenant la mise en contact de cette alimentation, à une température allant d'environ 200 à 350°C et pendant un temps suffisant pour convertir au moins une portion de cette alimentation en hydrocarbures alcanes, avec un catalyseur selon l'une quelconque des revendications 1 à 5.